Europäisches Patentamt

(19) European Patent Office

Office européen des brevets

(11) Publication number: 0 204 302
A2

## (12) EUROPEAN PATENT APPLICATION

(21) Application number: 86107491.2

(22) Date of filing: 03.06.86

(51) Int. Cl.⁴: **C 12 N 15/00**
C 12 P 21/02, A 61 K 37/02

(30) Priority: 03.06.85 US 740776
25.04.86 US 856615

(43) Date of publication of application:
10.12.86 Bulletin 86/50

(84) Designated Contracting States:
AT BE CH DE FR GB IT LI LU NL SE

(71) Applicant: MELOY LABORATORIES, INC.
6715 Electronic Drive
Springfield Virginia 22151(US)

(72) Inventor: Drohan, William N.
5232 Perth Court
Springfield Virginia(US)

(72) Inventor: Jaye, Michael C.
3017 South Second Street
Arlington Virginia(US)

(72) Inventor: Terranova, Victor P.
14404 Falling Leaf Drive
Gaiphersberg Maryland(US)

(74) Representative: Patentanwälte Grünecker, Kinkeldey,
Stockmair & Partner
Maximilianstrasse 58
D-8000 München 22(DE)

(54) Laminin and the production thereof.

(57) It is now possible to obtain large quantities of pure human laminin using the application of recombinant DNA technology to prepare cloning vehicles encoding for the laminin protein, and screening/isolating procedures for recovering the laminin. Also disclosed are expression vectors capable of expressing human laminin. The use of laminin for cosmetic purposes as well as in the treatment of damaged or degenerated epithelium is disclosed.

# LAMININ AND THE PRODUCTION THEREOF

This invention relates to the production of laminin. More particularly, this invention relates to human laminin and its recombinant DNA-directed synthesis, and laminin's use in the treatment of basement membrane damage or degeneration.

Laminin is a major component of basement membranes. Basement membranes are highly organized structures which support and regulate the passage of macromolecules across the epithelium. Laminin is a large glycoprotein (molecular weight of 1,000,000) which is synthesized by epithelial cells and localized to basement membranes.

As the major noncollagenous component of the basement membrane, laminin is believed to have several major functions. First, it regulates the formation of the basement membrane. Second, it influences the types of cells which abut the membrane by encouraging epithelial cell attachment and inhibiting fibroblast attachment. Third, laminin provides an environment for the continuing survival of epithelial cells by its attachment, growth

0204302

factor, and mitogenic properties toward epithelial cells. A review of the evidence supporting these postulated _in vivo_ properties and the mechanisms thereof can be found in "The Role of Laminin in Basement Membranes and in the Growth, Adhesion, and Differentiation of Cells," by Kleinman, et al., _The Role of Extracellular Matrix in Development_, pages 123-143, 1984; (Alan R. Liss, Inc., New York, New York) and the references contained therein.

In general, recombinant DNA techniques are known. See _Methods In Enzymology_, (Academic Press) Volumes 65 and 68 (1979); 100 and 101 (1983) and the references cited therein, all of which are incorporated herein by reference. An extensive technical discussion embodying most commonly used recombinant DNA methodologies can be found in Maniatis, et al., _Molecular Cloning_, Cold Spring Harbor Laboratory (1982). Genes coding for various polypeptides may be cloned by incorporating a DNA fragment coding for the polypeptide in a recombinant DNA vehicle, e.g., bacterial or viral vectors, and transforming a suitable host. This host is typically an _Escherichia coli_ (_E. coli_) cell line, however, depending upon the desired product, eukaryotic hosts may be utilized. Clones incorporating the recombinant vectors are isolated and may be grown and used to produce the desired polypeptide on a large scale.

Mixtures of mRNA from eukaryotic cells employing a series of three enzymatic reactions to synthesize double-stranded DNA copies of entire genes which are complementary to this mRNA mixture have been isolated. In the first reaction, mRNA is transcribed to form a single-stranded complementary DNA (cDNA) by an RNA-directed DNA polymerase, also called reverse transcriptase. Reverse transcriptase synthesizes DNA in the 5' -3' direction, utilizes deoxyribonucleoside

5'-triphosphates as precursors, and requires both a template and a primer strand, the latter of which must have a free 3'-hydroxyl terminus. Reverse transcriptase products, whether partial or complete copies of the mRNA template, often possess short, partially double-stranded hairpins ("loops") at their 3' termini. In the second reaction, these "hairpin loops" can be exploited as primers for DNA polymerases. Preformed DNA is required both as a template and as a primer in the action of DNA polymerase. The DNA polymerase requires the presence of a DNA strand having a free 3'-hydroxyl group, to which new nucleotides are added to extend the chain in the 5' - 3' direction. The products of such sequential reverse transcriptase and DNA polymerase reactions still possess a loop at one end. The apex of the loop or "fold-point" of the double-stranded DNA, which has thus been created, is substantially a single-strand segment. In the third reaction, this single-strand segment is cleaved with the single-strand specific nuclease S1 to generate a "blunt-end" duplex DNA segment. This general method is applicable to any mRNA mixture, and is described by Buell, et al., J. Biol. Chem., 253:2483 (1978).

The resulting double-stranded cDNA mixture (ds-cDNA) is inserted into cloning vehicles by any one of many known techniques, depending at least in part on the particular vehicle being used. Various insertion methods are discussed in considerable detail in Methods In Enzymology, 68:16-18, and the references cited therein.

Once the DNA segments are inserted, the cloning vehicle is used to transform a suitable host. These cloning vehicles usually impart an antibiotic resistance trait on the host. Such hosts are generally prokaryotic or eukaryotic cells. At this point, only a few of the

transformed or transfected hosts contain the desired cDNA. The sum of all transformed or transfected hosts constitutes a gene "library". The overall ds-cDNA library created by this method provides a representative sample of the coding information present in the mRNA mixture used as the starting material.

If an appropriate oligonucleotide sequence is available, it can be used to identify clones of interest in the following manner. Individual transformed or transfected cells are grown as colonies on nitrocellulose filter paper. These colonies are lysed; the DNA released is covalently attached to the filter paper by heating. The sheet is then incubated with a labeled oligonucleotide probe which is complementary to the structural gene of interest. The probe hybridizes with the cDNA for which it is complementary, and this is identified by autoradiography. The corresponding clones are characterized in order to identify one, or a combination of clones which contain all of the structural information for the desired protein. The nucleic acid sequence coding for the protein of interest is isolated and reinserted into an expression vector. The expression vector brings the cloned gene under the regulatory control of a specific prokaryotic or eukaryotic control element which allows the efficient expression (transcription and translation) of the cloned ds-cDNA. Thus, this general technique is only applicable to those proteins for which at least a portion of their amino acid or DNA sequence is known and for which an oligonucleotide probe is available. See, generally, Maniatis, et al., supra.

More recently, methods have been developed to identify specific clones by probing bacterial colonies with antibodies specific for the encoded protein of interest. This method can only be used with "expression vector" cloning vehicles since elaboration of the product protein is required. The structural gene is inserted into the vector adjacent to regulatory gene sequences that control expression of the protein. The cells are lysed, either by the vector or by chemical methods, and the protein detected by the specific antibody and a labeling system such as enzyme immunoassay. An example of this is the lambda $gt_{11}$ system described by Young and Davis, Proc. Nat'l. Acad. Sci. USA. 80:1194-1198 (1983) and Young and Davis, Science, 22:778 (1983).

**0204302**

The present invention has made it possible to provide readily available large quantities of human laminin or laminin subunits. This has been achieved with antibodies which react specifically with the laminin protein molecule, or subunits thereof, the application of recombinant DNA technology to preparing cloning vehicles encoding for the laminin protein, and screening/isolating procedures for recovering human laminin protein essentially free of other proteins of human origin.

Accordingly, the present invention provides human laminin or its subunits and fragments essentially free of other proteins of human origin. Characteristically, the laminin protein is glycosylated but may be in the unglycosylated form. Laminin is produced by recombinant DNA techniques in host cells or other self-replicating systems and is provided in essentially pure form.

The invention further provides replicable expression vectors incorporating a DNA sequence encoding human laminin and a self-replicating host cell system transformed or transfected thereby. The host system is usually a prokaryotic, e.g., E. coli, B. subtilis, or eukaryotic cells. The invention further provides a cDNA sequence which when correctly combined with an expression vector is capable of directing the synthesis of a polypeptide which is immunologically reactive with antibodies prepared against human laminin.

The human laminin is produced by a process which comprises (a) preparing a replicable expression vector capable of expressing the DNA sequence encoding human laminin or fragments thereof in a suitable host cell system; (b) transforming said host system to obtain a recombinant host system; (c) maintaining said recombinant host system under conditions permitting expression of said laminin encoding DNA sequence to produce human laminin protein; and (d) recovering said human laminin protein. Preferably, the laminin-encoding replicable expression vector is made by preparing a double-stranded complementary DNA (ds-cDNA) preparation representative of laminin mRNA and incorporating the ds-cDNA into replicable expression vectors. The preferred mode of recovering the human laminin comprises reacting the proteins expressed by the recombinant host system with a reagent composition comprising at least one binding protein specific for laminin.

Laminin or its fragments may be used as a therapeutic agent in the treatment of damaged or degenerated skin and other epithelial membranes.

The cloned cDNA coding for human laminin is also useful as a DNA probe for the specific identification and/or recovery of laminin DNA sequences from a mixture containing same.

Figure 1 illustrates a general procedure for enzymatic reactions to produce cDNA clones.

Figure 2 illustrates the production of a library containing DNA fragments inserted into lambda gt$_{11}$.

Figure 3 illustrates elaboration and purification of a human laminin cDNA clone from a lambda gt$_{11}$ recombinant library.

a.  Portion of the nitrocellulose filter, after incubation in HRP color development solution, showing a positive color reaction (Laml2).

b.  Portion of the nitrocellulose filter after replating and rescreening of a 4 mm diameter plug (From Step A., above) at the position of Laml2.

c.  Portion of the nitrocellulose filter after replating and rescreening of a 4 mm diameter plug (From Step B., above) at the position of Laml2.

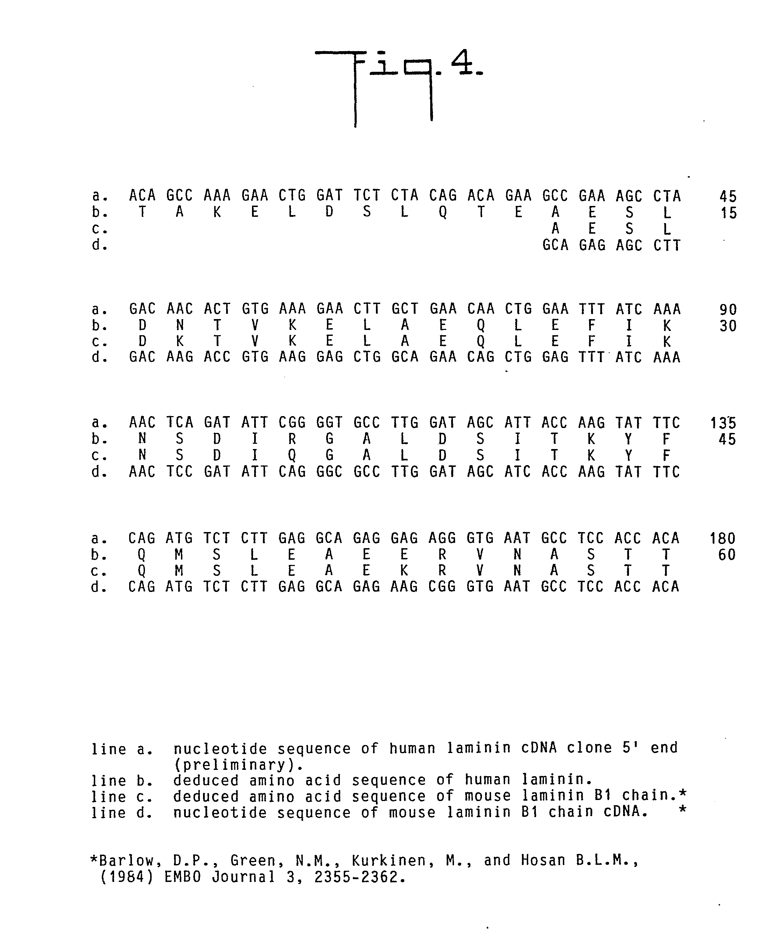

Figure 4 illustrates the nucleotide sequence obtained at the 5' end of Laml2.

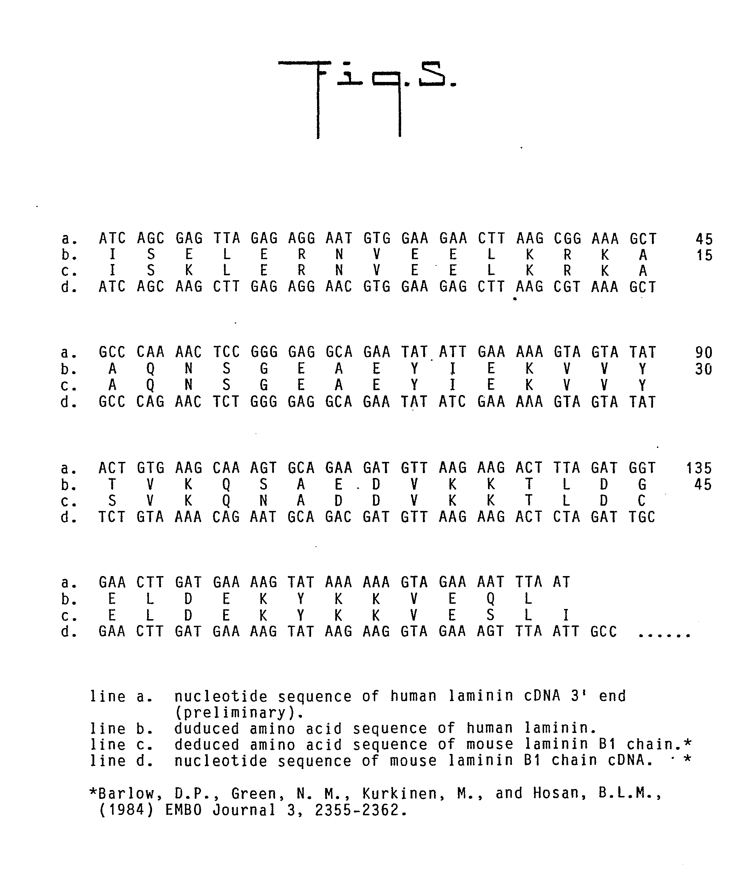

Figure 5 illustrates the nucleotide sequence obtained at the 3' end of Laml2.

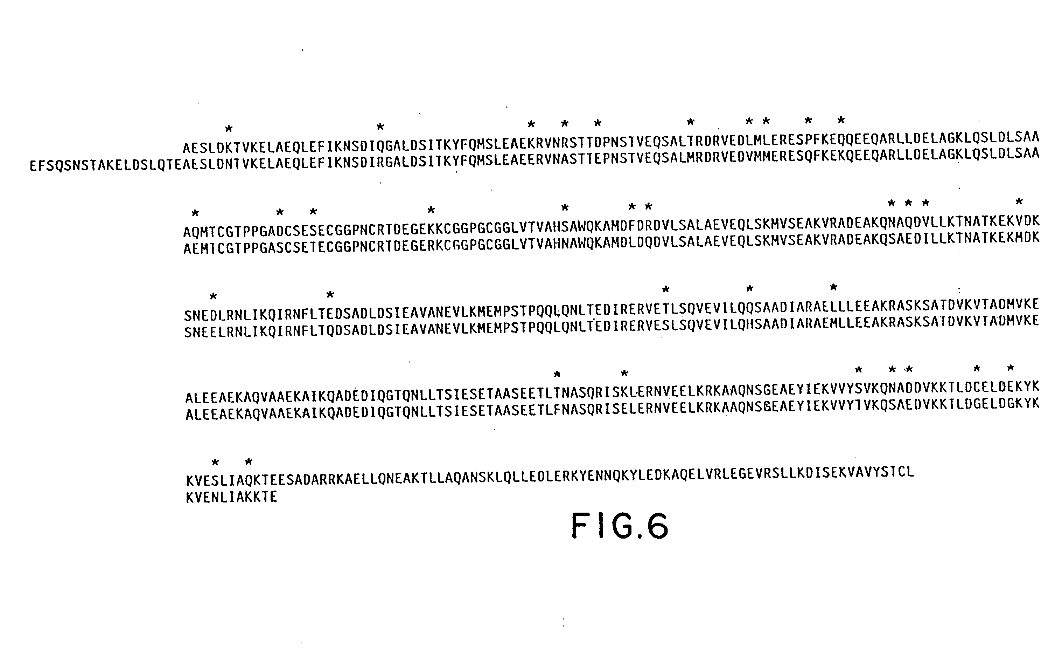

Figure 6 illustrates a comparison of mouse and human laminin Bl chains.  The amino acid sequence deduced from the cDNA sequence of a mouse laminin Bl chain is shown in the top line [Barlow, D.P., Green, N.M., Kurkinen, M., and Hogan, B.L.M., EMBO Journal, 3:2355-2362 (1984)].

The amino acid sequence deduced from the cDNA sequence of Lam12 is shown in the bottom line. Asterisks indicate non-homologous amino acids. A homology of 91.6% is observed between mouse and human laminin B1 chains.

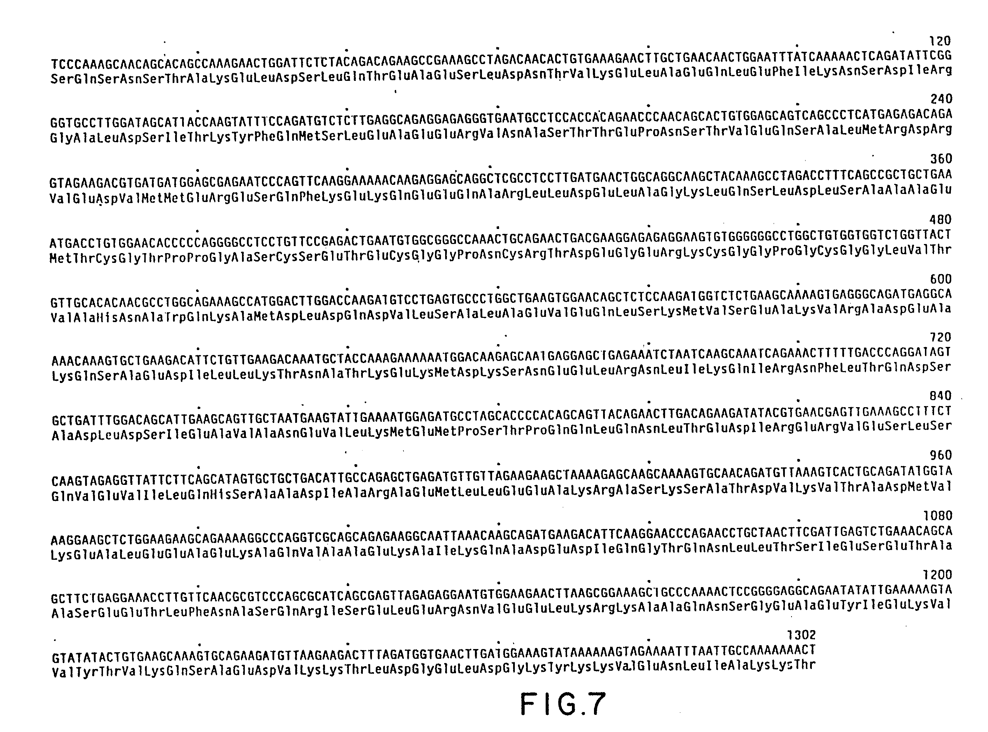

Figure 7 illustrates the complete nucleotide and deduced amino acid sequence of Lam12, a partial cDNA clone for the human B1 laminin chain. The nucleotide sequence at the 5' and 3' ends of Lam12 is shown in Figures 4 and 5. The complete nucleotide sequence of Lam 12, shown here, includes and joins the nucleotide sequences at the 5' and 3' ends of Lam12. Nucleotide sequence was determined by the chain termination method [Sanger et al., _Proc. Natl. Acad. Sci. USA_, 74:5463-5467 (1977)]. Nucleotide 146 in Figure 5 is erroneously reported as A, the actual nucleotide is G.

As used herein, "laminin" denotes human laminin or its subunits or fragments thereof produced by cell or cell-free culture systems, in bioactive forms having the capacity to influence cellular adhesion, growth, differentiation, and migration _in vivo_ as does laminin native to the human basement membrane and to react with antibodies raised to human laminin.

Different alleles of laminin may exist in nature. These variations may be characterized by difference(s) in the nucleotide sequence of the structural gene coding for proteins of identical biological function. In addition, the location and degree of glycosylation as well as other post-translational modifications may vary and will depend to a degree upon the nature of the host and environment in which the protein is produced. It is possible to produce analogs having single or multiple amino acid substitutions, deletions, additions, or replacements. All such allelic variations, modifications, and analogs resulting in derivatives of human laminin which retain the biologically active properties of native human laminin are included within the scope of this invention.

"Expression vectors" refer to vectors which are capable of transcribing and translating DNA sequences contained therein, where such sequences are linked to other regulatory sequences capable of effecting their expression. These expression vectors must be replicable in the host organisms or systems either as episomes, bacteriophage, or as an integral part of the chromosomal DNA. One expression vector which is particularly suitable for producing laminin is the bacteriophage, viruses which normally inhabit and replicate in bacteria. Particularly desirable phages for this purpose are the lambda $gt_{10}$ and $gt_{11}$ phage, described by Young and Davis, *supra*. Lambda $gt_{11}$ is a general recombinant DNA expression vector capable of producing polypeptides specified by the inserted DNA.

To minimize degradation, upon induction with a synthetic analogue of lactose (IPTG), foreign proteins or portions thereof are synthesized fused to the prokaryotic protein B-galactosidase. The use of host cells defective in protein degradation pathways may also increase the lifetime of novel proteins produced from the induced lambda $gt_{11}$ clones. Proper expression of foreign DNA in lambda $gt_{11}$ clones depends upon the proper orientation and reading frame of the inserted DNA with respect to the B-galactosidase promoter and ribosome binding site.

Another form of expression vector useful in recombinant DNA techniques is the plasmid - a circular, unintegrated (extra-chromosomal), double-stranded DNA loop. Any other form of expression vector which serves an equivalent function is suitable for use in the process of this invention.

Recombinant vectors and methodology disclosed herein are suitable for use in host cells covering a wide range of prokaryotic and eukaryotic organisms. Prokaryotics are preferred for the cloning of DNA sequences and in the construction of vectors. For example, *E. coli* K12 strain HB101 (ATCC No. 33694), is particularly useful. Of course, other microbial strains may be used. Vectors containing replication and control sequence which are derived from species compatible with the host cell or system are used in connection with these hosts. The vector ordinarily carries an origin of replication, as well as characteristics capable of providing phenotypic selection in transformed cells. For example, E. coli can be transformed using the vector pBR322, which contains genes for ampicillin and tetracycline resistance [Bolivar, et al., Gene, 2:95 (1977)].

These antibiotic resistance genes provide a means of identifying transformed cells. The expression vector may also contain control elements which can be used by the vector for expression of its own proteins. Common prokaryotic control elements used for expression of foreign DNA sequences in E. coli include the promoters and regulatory sequences derived from the B-galactosidase and tryptophan (trp) operons of E. coli, as well as the pR and pL promoters of bacteriophage lambda. Combinations of these elements have also been used (e.g., TAC, which is a fusion of the trp promoter with the lactose operator). Other promoters have also been discovered and utilized, and details concerning their nucleotide sequences have been published enabling a skilled worker to combine and exploit them functionally.

In addition to prokaryotes, eukaryotic microbes, such as yeast cultures, may also be used. Saccharomyces cerevisiae, or common baker's yeast, is the most commonly used among eukaryotic microorganisms, although a number of other strains are commonly available. Suitable promoting sequences in yeast vectors include the promoters of 3-phosphoglycerate kinase or other glycolytic enzymes. Suitable expression vectors may contain termination signals which provide for the polyadenylation and termination of the cloned gene's mRNA. Any vector containing a yeast-compatible promoter, origin of replication, and appropriate termination sequence is suitable for expression of laminin.

In addition to microorganisms, cultures of cells derived from multicellular organisms may also be used as hosts. In principle, any such cell culture is workable, whether from a vertebrate or invertebrate source. However, interest has been greatest in vertebrate cells, and propagation of vertebrate cells in culture (tissue culture) has become a routine procedure in recent years. Examples of such useful hosts are the VERO, HeLa, mouse C127, Chinese hamster ovary (CHO), Wl38, BHK, COS-7, and MDCK cell lines. Expression vectors for such cells ordinarily include an origin of replication, a promoter located in front of the gene to be expressed, along with any necessary ribosome binding sites, RNA splice sites, polyadenylation site, and transcriptional terminatory sequence.

For use in mammalian cells, the control functions on the expression vectors are often provided by viral material. For example, commonly used promoters are derived from polyoma, Adenovirus 2, and most frequently, Simian Virus 40 (SV40). Further, it is also possible, and often desirable, to utilize promoter or control sequence naturally associated with the desired gene sequence, provided such control sequences are compatible with the host system. To increase the rate of transcription, eukaryotic enhancer sequences can also be added to the construction. These sequences can be obtained from a variety of animal cells or oncorna viruses such as the mouse sarcoma virus.

An origin of replication may be provided either by construction of the vector to include an exogenous origin, such as that provided by SV40 or other viral sources, or may be provided by the host cell chromosomal replication mechanism. If the vector is integrated into the host cell chromosome, the latter is sufficient.

Host cells can prepare human laminin proteins which can be of a variety of chemical compositions. The protein is produced having methionine as its first amino acid (present by virtue of the ATG start signal codon naturally existing at the origin of the structural gene or inserted before a segment of the structural gene). The protein may also be intra- or extracellularly cleaved, giving rise to the amino acid which is found naturally at the amino terminus of the protein. The protein may be produced together with either its signal polypeptide or a conjugated protein other than the conventional signal polypeptide, the signal polypeptide of the conjugate being specifically cleavable in an intra- or extracellular environment. Finally, laminin may be produced by direct expression in mature form without the necessity of cleaving away any extraneous polypeptide.

Recombinant host cells which have been transformed with vectors constructed using recombinant DNA techniques. As defined herein, laminin is produced as a consequence of this transformation. Laminin or its subunits or fragments thereof produced by such cells are referred to as "recombinant laminin".

The procedures below are but some of a wide variety of well established procedures to produce specific reagents useful in this invention. The general procedure for obtaining a messenger RNA (mRNA) mixture is to prepare an extract from a tissue sample or to culture cells producing the desired protein, and to extract the mRNA by a process such as that disclosed by Chirgwin, et al., Biochemistry, 18:5294 (1979). The mRNA is enriched for poly(A) mRNA-containing material by chromatography on oligo (dT) cellulose or poly(U) Sepharose, followed by elution of the poly(A) containing mRNA-enriched fraction.

The above poly(A) containing mRNA-enriched fraction is used to synthesize a single-strand complementary cDNA (ss-cDNA) using reverse teanscriptase. As a consequence of DNA synthesis, a hairpin loop is formed at the 3' end of the DNA which will initiate second strand DNA synthesis. Under appropriate conditions, this hairpin loop is used to effect synthesis of the second strand in the presence of DNA polymerase and nucleotide triphosphates.

The resultant double-strand cDNA (ds-cDNA) is inserted into the expression vector by any one of many known techniques. In general, methods, etc., can be found in Maniatis, supra, and Methods In Enzymology, Vol. 65 and 68 (1980); and Vol. 100 and 101 (1983). In general, the vector is linearized by at least one restriction endonuclease, which will produce at least two blunt or cohesive ends. The ds-cDNA is ligated with or joined to the vector insertion site.

If prokaryotic cells or other cells which contain substantial cell wall material are employed, the most common method of transformation with the expression vector is calcium chloride pretreatment as described by Cohen, R.N., et al., Proc. Nat'l. Sci. USA, 69:2110 (1972). If cells without cell wall barriers are used as host cells, transfection is carried out by the calcium phosphate precipitation method described by Graham and Van der Eb, Virology, 62:456 (1973). Other methods for introducing DNA into cells such as nuclear injection or protoplast fusion, have also been successfully used. The organisms are then cultured on selective media and proteins for which the expression vector encodes are produced.

Clones containing part or the entire gene for laminin are identified with specific binding protein directed against part or all of the laminin protein. The specific binding protein may preferably be a primary antibody. For example, these primary antibodies include both a polyclonal antibody or a monoclonal antibody. This method of identification requires that the ds-cDNA be inserted into a vector containing appropriate regulatory nucleic acid sequences adjacent to the insertion site. These regulatory sequences initiate transcription and translation of those ds-cDNA molecules inserted in the vector. Those clones cointaining laminin cDNA sequences correctly positioned relative to the regulatory sequences synthesize part or all of the laminin protein. Such clones are detected using appropriately specific antibodies. Such a cloning system is the lambda $gt_{11}$ system first described by Young and Davis, supra.

Clones containing the entire sequence of laminin are identified using as probe the cDNA insert of the laminin recombinant isolated during immunoassay screening of the recombinant lambda $gt_{11}$ human endothelial cDNA library. Nucleotide sequencing techniques are used to determine the sequence of amino acids encoded by the cDNA fragments. This information may be used to determine the identity of cDNA clones as specific for human laminin by comparison to the amino acid sequence of isolated laminin chains. Alternatively, identification may be confirmed by employing techniques such as Northern blot analysis and hybrid-selected translation or by comparison to laminin clones isolated from other species, e.g., mouse and rat.

EXAMPLE

A. Preparation of Total RNA

Total RNA (messenger, ribosomal, and transfer) was extracted from fresh human umbilical vein endothelial cells essentially as described by Chirgwin, supra, (1979). Cell pellets were homogenized in 5 volumes of a solution containing 4 M guanidine thiocyanate, 25 mM sodium citrate at pH 7.0, 0.5% N-laurylsarcosine, 0.1 M 2-mercaptoethanol, and 0.2% Antifoam A (Sigma Chemical Co., St. Louis, MO). The homogenate was centrifuged at 6,000 rpm in a Sorvall GSA rotor for 15 minutes at $10^{o}C$. The supernatant fluid was adjusted to pH 5.0 by addition of acetic acid and the RNA precipitated by 0.75 volumes of ethanol at $-20^{o}C$. for two hours. RNA was collected by centrifugation and dissolved in 7.5 M guanidine hydrochloride containing 2 mM sodium citrate and 5 mM dithiothreitol. Following two additional precipitations using 0.5 volumes of ethanol, the residual guanidine hydrochloride was extracted from the precipitate with absolute ethanol. RNA was dissolved in sterile water, insolvable material removed by centrifugation, and the pellets were re-extracted with water. The RNA was adjusted to 0.2 M potassium acetate and precipitated by addition of 2.5 volumes of ethanol at $-20^{o}C$. overnight.

B. Preparation of Poly(A)-containing RNA

The total RNA precipitate, prepared as described above, was dissolved in 20 mM Hepes buffer (pH 7.2) containing 10 mM EDTA and 1% SDS, heated at $65^{o}C$. for 10 minutes, then quickly cooled to $25^{o}C$. The RNA solution was then diluted with an equal volume of water and NaCl was added to bring the final concentration to 300 mM NaCl. Samples containing up to 240 $A_{260}$ units of RNA were chromotographed on poly(U)-sepharose using standard procedures. Poly(A)-containing RNA was eluted with 70% formamide containing 1 mM Hepes buffer (pH 7.2), and 2mM EDTA. The eluate was adjusted to 0.24 M NaCl and the RNA was precipitated by 2.5 volumes of ethanol at -20°C.

C.  Construction of cDNA Clones in Lambda gt$_{11}$

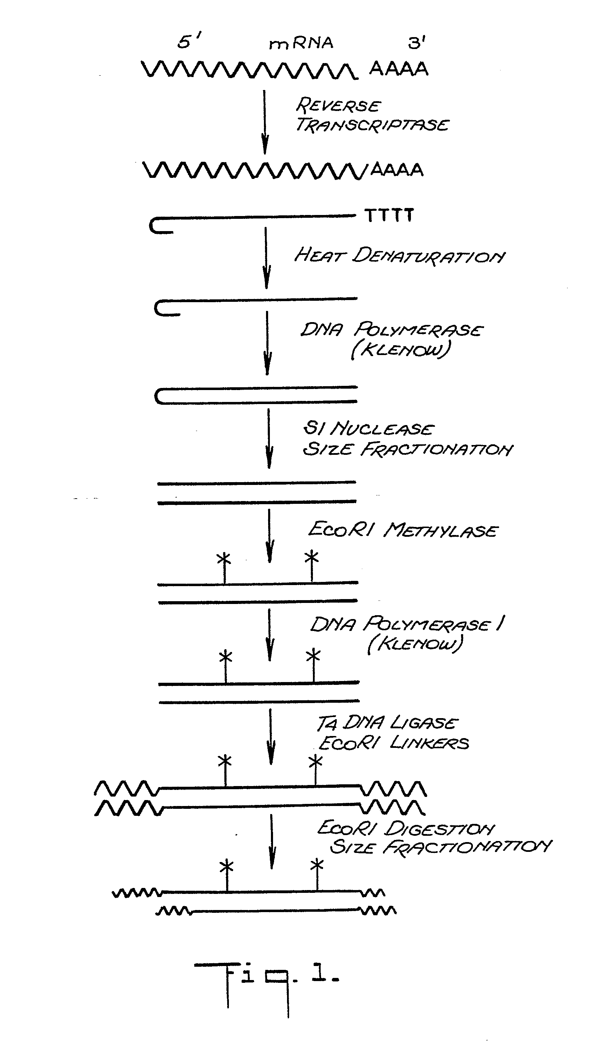

The procedure followed for the enzymatic reaction is shown in Figure 1. The mRNA (20 ug) was copied into ds-cDNA with reverse transcriptase and DNA polymerase I exactly as described by Buell, et al., supra, and Wilkensen, et al., J. Biol. Chem., 253:2483 (1978). The ds-cDNA was desalted on Sephadex G-50 and the void volume fractions further purified on an Elutip-D column, (Schleicher & Schuell, Keene, NH), following the manufacturer's directions. The ds-cDNA was made blunt-ended by incubation with Sl nuclease, Ricca, et al, J. Biol. Chem. 256 : 10362 (1981). The reaction mixture consisted of 0.2 M sodium acetate (pH 4.5), 0.4 sodium chloride, 2.5 mM zinc acetate and 0.1 unit of Sl nuclease per ng of ds-cDNA, made to a final reaction volume of 100 $\mu$l. The ds-cDNA was incubated at 37°C. for one hour, extracted with phenol:chloroform, and then desalted on a Sephadex G-50 column as described above.

The double-stranded cDNA was then treated with Eco RI methylase and DNA polymerase I (Klenow) using reaction conditions described in Maniatis, Molecular Cloning, supra. The cDNA was again desalted on Sephadex G-50 as described above and then ligated to 0.5 $\mu$g of phosphorylated Eco RI linkers using T$_4$ DNA ligase (Maniatis, supra). The mixture was then cleaved with Eco RI and fractionated on an 8% acrylamide gel in Tris-Borate buffer (Maniatis, supra). DNA with a size greater than 1 kilobase was eluted from the gel and recovered by binding to an Elutip-D column, eluted with 1 M NaCl and then collected by ethanol precipitation.

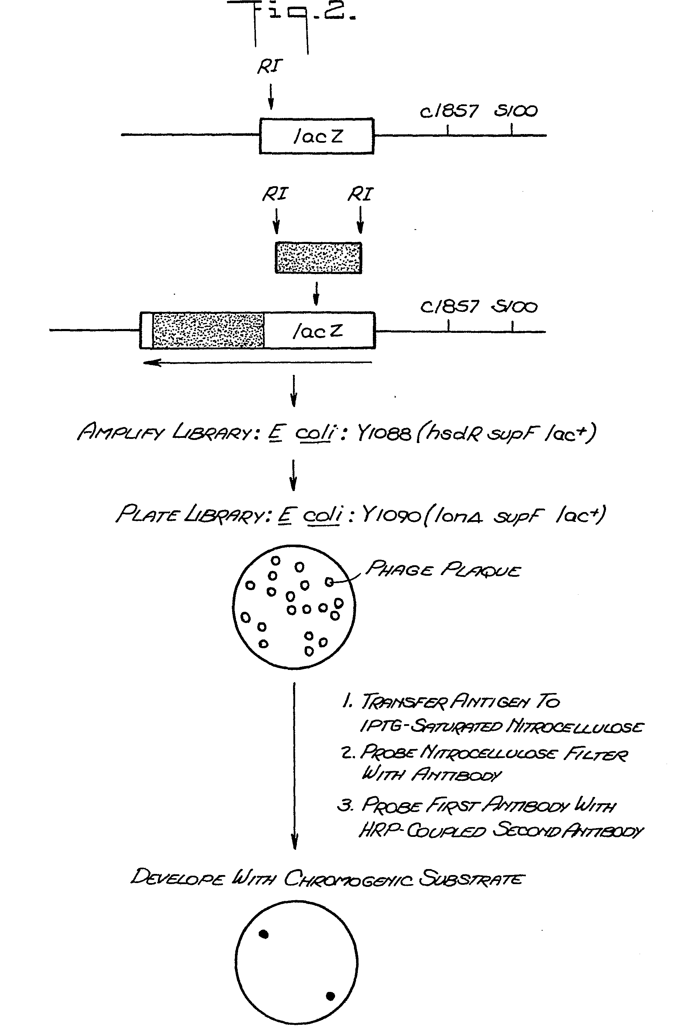

As shown in Figure 2 the DNA fragments were then inserted into Eco RI cleaved and phosphatase-treated lambda $gt_{11}$, using $T_4$ DNA ligase. A library of approximately five hundred thousand recombinant phage was produced. The library was amplified by producing plate stocks at $4^O$C. on E. coli Y1083 [supE SupF metB trpR hsdR$^-$ hsdm$^+$ tonA21 strA$\triangle$lacU169 proc::Tn5 (pMC9)]. Amplification procedures are described in Maniatis, supra. Important features of this strain, described by Young and Davis, include (1) supF (required suppression of the phage amber mutation in the S gene), (2). hsdR$^-$ hsdM$^+$ (necessary to prevent restriction of foreign DNA prior to host modification, (3)$\triangle$ lacU169 (a deletion of the lac operon which reduces host-phage recombination and which is necessary to distinguish between lambda $gt_{11}$ recombinants and non-recombinants), and (4) pMC9 (a lacI-bearing pBR322 derivative which represses, in the absence of an inducer, the expression of foreign genes that may be detrimental to phage and/or cell growth).

D.   Identification of Clones Containing Laminin Sequence

To screen the library for laminin antigenic determinant-producing clones, 500,000 lambda $gt_{11}$ recombinant phage were plated on a lawn of E. coli Y1090 [$\triangle$lacU169 proA$^+$$\triangle$lon araD139 strA supF (trpC22::Tn10) (pMC9)] and incubated at $42^O$C. for 4 hours. This host is deficient in the lon protease, thereby reducing the degradation of expressed foreign protein. A nitrocellulose filter, previously saturated with 10 mM isopropyl thio-B-d-galactopyranoside (IPTG) and dried, was overlaid on the plates. The plates were then incubated at $37^O$C. overnight. Since IPTG is an inducer of lacZ transcription, the expression of foreign DNA inserts in

lambda gt$_{11}$ is under common control with lacZ transcription, and, as such, is also induced.  The position of the filter was marked with a needle, the filter removed, washed in TBS buffer (20 mM Tris, pH 7.5, 500 mM NaCl), and incubated in TBS plus 3% gelatin for 60 minutes at room temperature.

The filter was then incubated at room temperature overnight in a 1:100 dilution of a rabbit polyclonal antibody directed against rat laminin in a buffer consisting of 1% gelatin in TBS.  After 2 thirty-minute washes with TBS at room temperature, 20 ml of a 1:2000 dilution of horseradish peroxidase (HRP) conjugated goat anti-rabbit antisera (Bio-Rad, Richmond, CA) was added. The filters were then incubated for 2 hours at room temperature, and then washed 2 times for thirty minutes in TBS.  The filters were then incubated at room temperature in HRP color development solution as described in the Bio-Rad (<u>supra</u>) accompanying literature.

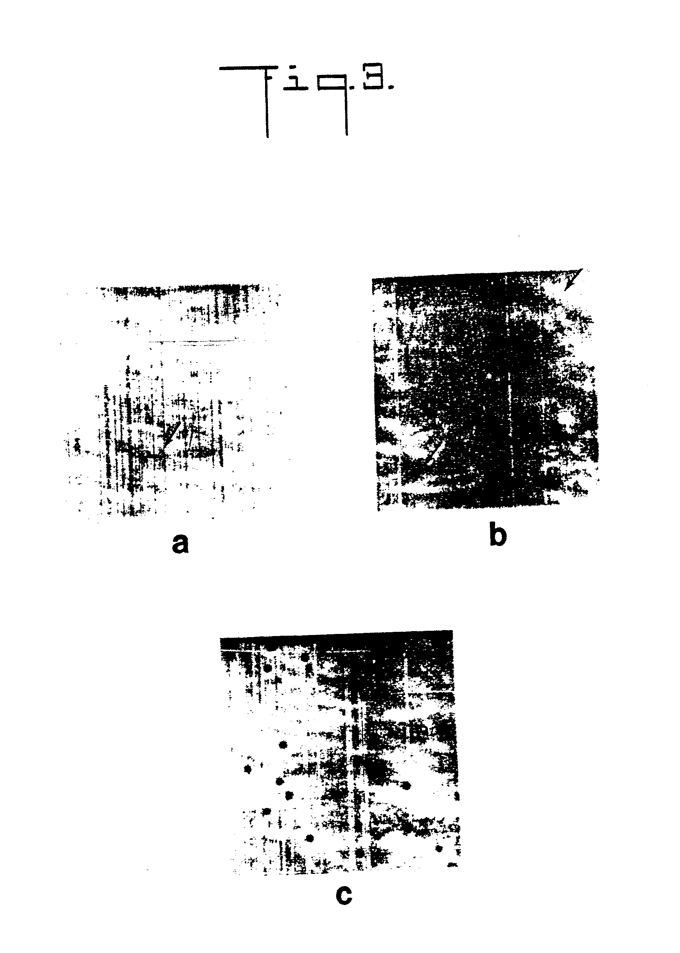

A 4 mm diameter plug at the position of each of the color development signals (Fig. 3) was removed from the plates and incubated in 10 mM Tris HCl, pH 7.5, and 10 mM MgSO$_4$ for one hour.  Approximately $10^3$ plaque-forming units (PFU) were replated on 90 mm plates and rescreened as described above.  This replating and rescreening process was repeated until all plaques on the plate produced a signal.

The cDNA insert from Laml2 was excised using the restricton enzyme Eco RI.  The cDNA insert (approximately 1.4 Kb in length) was subcloned into the Eco RI site of pUC9.  Recombinant plasmids are called pMJ5.  This construction was used to transform an <u>E.coli</u> host and the resulting transformant was deposited with the American Type Culture Collection, 12301 Parklawn Drive, Rockville, MD 20852 on <u>May 9, 1986</u> in accordance with the provisions of the Budapest treaty. After viability testing, the accession number ATCC 67112 was assigned. The cDNA insert was also subcloned into the Eco RI site of M13mp11. The recombinant phage were isolated and subjected to nucleic acid sequence analysis as described by Sanger. [Sanger, et al., Proc. Natl. Acad. Sci. USA. 74:5463 (1977)].

The sequences obtained at the 5' and 3' ends of Laml2 are shown in Figures 4 & 5, respectively at Lines a. Lines b show the amino acid sequence of laminin, which can be deduced from the nucleotide sequence. Lines c show the amino acid sequence deduced from the sequencing of a mouse laminin B chain cDNA, and Lines d show the nucleic acid sequence of the mouse laminin cDNA [Barlow, D.P., N.M. Green, M. Kurkinen, and B.L.M. Hogan, (1984) EMBO Journal, 3:2355-2362]. The data indicate that Laml2 cDNA encodes a COOH-terminal portion of the human laminin B1 chain. The amino acid sequences deduced from the human and mouse clones share 92% homology, while the nucleotide sequences share 90% homology. These homologies show that Laml2 is a cDNA clone of the human laminin B chain, and suggest extreme conservation of this protein in nature.

A pharmaceutical or cosmetic preparation containing the laminin of this invention may be prepared according to methods well known in the art for the treatment of epithelial damage or degeneration. The therapeutic preparation which contains the laminin of this invention may be conveniently mixed with a non-toxic pharmaceutical organic carrier or a non-toxic pharmaceutical inorganic carrier. The pharmaceutical carrier of choice may take the form of a gel or cream. In general, the formulations of this invention utilize only an effective amount of laminin which for pharmaceutical preparations is somewhat higher than for cosmetic preparations. A therapeutically effective amount of laminin may be about 1 to 5 micrograms per milliliter of carrier.

Thus, this example describes experimental procedures which provide human laminin essentially free of other proteins of human original from a human tissue extract containing laminin-specific mRNA in a heterogeneous mRNA population. It should be appreciated that the present invention is not to be construed as being limited by the illustrative embodiment. It is possible to produce still other embodiments without departing from the inventive concepts herein disclosed. Such embodiments are within the ability of those skilled in the art.

WHAT IS CLAIMED IS:

1. A cDNA sequence which when correctly combined with an expression vector is capable of directing the synthesis of a polypeptide which is immunologically reactive with antibodies prepared against human laminin.

2. The cDNA sequence according to Claim 1 having the polynucleotide sequence according to Figure 7.

3. The expression vector according to Claim 1 or 2 derived from a vector selected from the group consisting of lambda $gt_{10}$, lambda $gt_{11}$, and pBR322.

4. The cDNA sequence according to Claim 1 or 2 which when correctly combined with a cloning vector forms a self-replicating recombinant system upon transformation of an appropriate host.

5. The self-replicating recombinant system according to Claim 4 wherein the host is E. coli.

6. The self-replicating recombinant system according to Claim 4 or 5 having the identifying characteristics of ATCC 67112.

7. A process for preparing human laminin from a heterogeneous mRNA mixture containing mRNA for said protein, comprising preparing a heterogeneous ds-cDNA population complementary to a heterogeneous mRNA mixtures, incorporating said ds-cDNA into a bacteriophage direct expression vector, providing a bacteriophage direct expression vector capable of incorporating said ds-cDNA population, expressing human laminin from said ds-cDNA-containing bacteriophage, and isolating and recovering human laminin.

8. The process according to Claim 7 wherein recovering said human laminin comprises reaction of the proteins expressed by the recombinant host system with a reagent composition comprising at least one binding protein specific for laminin.

9. The process according to Claim 8 wherein the specific binding protein is a primary antibody.

10. The process according to Claims 8 or 9 wherein the antibody is polyclonal antibody.

11. The process according to Claims 8 or 9 wherein the antibody is a monoclonal antibody.

12. Human laminin or fragments thereof esentially free of other proteins of human origin, in glycosylated or unglycosylated form, when made by the process of Claim 7.

13. A composition comprising a therapeutically effective amount of human laminin in a mixture with an acceptable carrier.

14. The use of laminin according to Claim 13 for treatment of epithelial damage or degeneration or for the preparation of pharmaceutical or cosmetic compositions useful in such treatment.

15. An essentially pure laminin fragment having the deduced amino acid sequence illustrated in Figure 7.

Fig. 1.

0204302

# Fig.2.

RI

c1857 S100

/acZ

RI          RI

c1857 S100

/acZ

AMPLIFY LIBRARY: E coli: Y1088 (hsdR supF lac⁺)

PLATE LIBRARY: E coli: Y1090 (lonΔ supF lac⁺)

PHAGE PLAQUE

1. TRANSFER ANTIGEN TO
   IPTG-SATURATED NITROCELLULOSE

2. PROBE NITROCELLULOSE FILTER
   WITH ANTIBODY

3. PROBE FIRST ANTIBODY WITH
   HRP-COUPLED SECOND ANTIBODY

DEVELOPE WITH CHROMOGENIC SUBSTRATE

0204302

Fig.3.

a

b

c

# Fig.4.

```
a.  ACA GCC AAA GAA CTG GAT TCT CTA CAG ACA GAA GCC GAA AGC CTA    45
b.   T   A   K   E   L   D   S   L   Q   T   E   A   E   S   L     15
c.                                               A   E   S   L
d.                                              GCA GAG AGC CTT


a.  GAC AAC ACT GTG AAA GAA CTT GCT GAA CAA CTG GAA TTT ATC AAA    90
b.   D   N   T   V   K   E   L   A   E   Q   L   E   F   I   K     30
c.   D   K   T   V   K   E   L   A   E   Q   L   E   F   I   K
d.  GAC AAG ACC GTG AAG GAG CTG GCA GAA CAG CTG GAG TTT ATC AAA


a.  AAC TCA GAT ATT CGG GGT GCC TTG GAT AGC ATT ACC AAG TAT TTC   135
b.   N   S   D   I   R   G   A   L   D   S   I   T   K   Y   F     45
c.   N   S   D   I   Q   G   A   L   D   S   I   T   K   Y   F
d.  AAC TCC GAT ATT CAG GGC GCC TTG GAT AGC ATC ACC AAG TAT TTC


a.  CAG ATG TCT CTT GAG GCA GAG GAG AGG GTG AAT GCC TCC ACC ACA   180
b.   Q   M   S   L   E   A   E   E   R   V   N   A   S   T   T     60
c.   Q   M   S   L   E   A   E   K   R   V   N   A   S   T   T
d.  CAG ATG TCT CTT GAG GCA GAG AAG CGG GTG AAT GCC TCC ACC ACA
```

line a.  nucleotide sequence of human laminin cDNA clone 5' end
         (preliminary).
line b.  deduced amino acid sequence of human laminin.
line c.  deduced amino acid sequence of mouse laminin B1 chain.*
line d.  nucleotide sequence of mouse laminin B1 chain cDNA.   *

*Barlow, D.P., Green, N.M., Kurkinen, M., and Hosan B.L.M.,
 (1984) EMBO Journal 3, 2355-2362.

# Fig.5.

```
a.  ATC AGC GAG TTA GAG AGG AAT GTG GAA GAA CTT AAG CGG AAA GCT    45
b.   I   S   E   L   E   R   N   V   E   E   L   K   R   K   A     15
c.   I   S   K   L   E   R   N   V   E   E   L   K   R   K   A
d.  ATC AGC AAG CTT GAG AGG AAC GTG GAA GAG CTT AAG CGT AAA GCT


a.  GCC CAA AAC TCC GGG GAG GCA GAA TAT ATT GAA AAA GTA GTA TAT    90
b.   A   Q   N   S   G   E   A   E   Y   I   E   K   V   V   Y     30
c.   A   Q   N   S   G   E   A   E   Y   I   E   K   V   V   Y
d.  GCC CAG AAC TCT GGG GAG GCA GAA TAT ATC GAA AAA GTA GTA TAT


a.  ACT GTG AAG CAA AGT GCA GAA GAT GTT AAG AAG ACT TTA GAT GGT   135
b.   T   V   K   Q   S   A   E   D   V   K   K   T   L   D   G     45
c.   S   V   K   Q   N   A   D   D   V   K   K   T   L   D   C
d.  TCT GTA AAA CAG AAT GCA GAC GAT GTT AAG AAG ACT CTA GAT TGC


a.  GAA CTT GAT GAA AAG TAT AAA AAA GTA GAA AAT TTA AT
b.   E   L   D   E   K   Y   K   K   V   E   Q   L
c.   E   L   D   E   K   Y   K   K   V   E   S   L   I
d.  GAA CTT GAT GAA AAG TAT AAG AAG GTA GAA AGT TTA ATT GCC    ......
```

line a.  nucleotide sequence of human laminin cDNA 3' end (preliminary).
line b.  duduced amino acid sequence of human laminin.
line c.  deduced amino acid sequence of mouse laminin B1 chain.*
line d.  nucleotide sequence of mouse laminin B1 chain cDNA. · *

*Barlow, D.P., Green, N. M., Kurkinen, M., and Hosan, B.L.M., (1984) EMBO Journal 3, 2355-2362.

```
       *               *              *  *  *      *    * *   *  *
     AESLDKTVKELAEQLEFIKNSDIQGALDSITKYFQMSLEAEKRVNRSTTDPNSTVEQSALTRDRVEDLMLERESPFKEQQEEQARLLDELAGKLQSLDLSAA
EFSQSNSTAKELDSLQTEAESLDNTVKELAEQLEFIKNSDIRGALDSITKYFQMSLEAEERVNASTTEPNSTVEQSALMRDRVEDVMMERESQFKEKQEEQARLLDELAGKLQSLDLSAA

     *        *   *          *              *       * *                      * * *           *
     AQMTCGTPPGADCSESECGGPNCRTDEGEKKCGGPGCGGLVTVAHSAWQKAMDFDRDVLSALAEVEQLSKMVSEAKVRADEAKQNAQDVLLKTNATKEKVDK
     AEMTCGTPPGASCSETECGGPNCRTDEGERKCGGPGCGGLVTVAHNAWQKAMDLDQDVLSALAEVEQLSKMVSEAKVRADEAKQSAEDILLKTNATKEKMDK

     *               *                       *          *        *
     SNEDLRNLIKQIRNFLTEDSADLDSIEAVANEVLKMEMPSTPQQLQNLTEDIRERVETLSQVEVILQQSAADIARAELLLEEAKRASKSATDVKVTADMVKE
     SNEELRNLIKQIRNFLTQDSADLDSIEAVANEVLKMEMPSTPQQLQNLTEDIRERVESLSQVEVILQHSAADIARAEMLLEEAKRASKSATDVKVTADMVKE

                                          *       *                          * * *       *   *
     ALEEAEKAQVAAEKAIKQADEDIQGTQNLLTSIESETAASEETLTNASQRISKLERNVEELKRKAAQNSGEAEYIEKVVYSVKQNADDVKKTLDCELDEKYK
     ALEEAEKAQVAAEKAIKQADEDIQGTQNLLTSIESETAASEETLFNASQRISELERNVEELKRKAAQNSGEAEYIEKVVYTVKQSAEDVKKTLDGELDGKYK

     *   *
     KVESLIAQKTEESADARRKAELLQNEAKTLLAQANSKLQLLEDLERKYENNQKYLEDKAQELVRLEGEVRSLLKDISEKVAVYSTCL
     KVENLIAKKTE
```

# FIG.6

```
TCCCAAAGCAACAGCACAGCCAAAGAACTGGATTCTCTACAGACAGAAGCCGAAAGCCTAGACAACACTGTGAAAGAACTTGCTGAACAACTGGAATTTATCAAAAACTCAGATATTCGG
SerGlnSerAsnSerThrAlaLysGluLeuAspSerLeuGlnThrGluAlaGluSerLeuAspAsnThrValLysGluLeuAlaGluGlnLeuGluPheIleLysAsnSerAspIleArg

GGTGCCTTGGATAGCATTACCAAGTATTTCCAGATGTCTCTTGAGGCAGAGGAGAGGGTGAATGCCTCCACCACAGAACCCAACAGCACTGTGGAGCAGTCAGCCCTCATGAGAGACAGA
GlyAlaLeuAspSerIleThrLysTyrPheGlnMetSerLeuGluAlaGluGluArgValAsnAlaSerThrThrGluProAsnSerThrValGluGlnSerAlaLeuMetArgAspArg

GTAGAAGACGTGATGATGGAGCGAGAATCCCAGTTCAAGGAAAAACAAGAGGAGCAGGCTCGCCTCCTTGATGAACTGGCAGGCAAGCTACAAAGCCTAGACCTTTCAGCCGCTGCTGAA
ValGluAspValMetMetGluArgGluSerGlnPheLysGluLysGlnGluGluGlnAlaArgLeuLeuAspGluLeuAlaGlyLysLeuGlnSerLeuAspLeuSerAlaAlaAlaGlu

ATGACCTGTGGAACACCCCCAGGGGCCTCCTGTTCCGAGACTGAATGTGGCGGGCCAAACTGCAGAACTGACGAAGGAGAGAGGAAGTGTGGGGGGCCTGGCTGTGGTGGTCTGGTTACT
MetThrCysGlyThrProProGlyAlaSerCysSerGluThrGluCysGlyGlyProAsnCysArgThrAspGluGlyGluArgLysCysGlyGlyProGlyCysGlyGlyLeuValThr

GTTGCACACAACGCCTGGCAGAAAGCCATGGACTTGGACCAAGATGTCCTGAGTGCCCTGGCTGAAGTGGAACAGCTCTCCAAGATGGTCTCTGAAGCAAAAGTGAGGGCAGATGAGGCA
ValAlaHisAsnAlaTrpGlnLysAlaMetAspLeuAspGlnAspValLeuSerAlaLeuAlaGluValGluGlnLeuSerLysMetValSerGluAlaLysValArgAlaAspGluAla

AAACAAAGTGCTGAAGACATTCTGTTGAAGACAAATGCTACCAAAGAAAAAATGGACAAGAGCAATGAGGAGCTGAGAAATCTAATCAAGCAAATCAGAAACTTTTTGACCCAGGATAGT
LysGlnSerAlaGluAspIleLeuLeuLysThrAsnAlaThrLysGluLysMetAspLysSerAsnGluGluLeuArgAsnLeuIleLysGlnIleArgAsnPheLeuThrGlnAspSer

GCTGATTTGGACAGCATTGAAGCAGTTGCTAATGAAGTATTGAAAATGGAGATGCCTAGCACCCCACAGCAGTTACAGAACTTGACAGAAGATATACGTGAACGAGTTGAAAGCCTTTCT
AlaAspLeuAspSerIleGluAlaValAlaAsnGluValLeuLysMetGluMetProSerThrProGlnGlnLeuGlnAsnLeuThrGluAspIleArgGluArgValGluSerLeuSer

CAAGTAGAGGTTATTCTTCAGCATAGTGCTGCTGACATTGCCAGAGCTGAGATGTTGTTAGAAGAAGCTAAAAGAGCAAGCAAAAGTGCAACAGATGTTAAAGTCACTGCAGATATGGTA
GlnValGluValIleLeuGlnHisSerAlaAlaAspIleAlaArgAlaGluMetLeuLeuGluGluAlaLysArgAlaSerLysSerAlaThrAspValLysValThrAlaAspMetVal

AAGGAAGCTCTGGAAGAAGCAGAAAAAGGCCCAGGTCGCAGCAGAGAAGGCAATTAAACAAGCAGATGAAGACATTCAAGGAACCCAGAACCTGCTAACTTCGATTGAGTCTGAAACAGCA
LysGluAlaLeuGluGluAlaGluLysAlaGlnValAlaAlaGluLysAlaIleLysGlnAlaAspGluAspIleGlnGlyThrGlnAsnLeuLeuThrSerIleGluSerGluThrAla

GCTTCTGAGGAAACCTTGTTCAACGCGTCCCAGCGCATCAGCGAGTTAGAGAGGAATGTGGAAGAACTTAAGCGGAAAGCTGCCCAAAACTCCGGGGAGGCAGAATATATTGAAAAAGTA
AlaSerGluGluThrLeuPheAsnAlaSerGlnArgIleSerGluLeuGluArgAsnValGluGluLeuLysArgLysAlaAlaGlnAsnSerGlyGluAlaGluTyrIleGluLysVal

GTATATACTGTGAAGCAAAGTGCAGAAGATGTTAAGAAGACTTTAGATGGTGAACTTGATGGAAAGTATAAAAAAGTAGAAAATTTAATTGCCAAAAAAACT
ValTyrThrValLysGlnSerAlaGluAspValLysLysThrLeuAspGlyGluLeuAspGlyLysTyrLysLysValGluAsnLeuIleAlaLysLysThr
```

# FIG.7